# EUROPEAN PATENT APPLICATION

(11) **EP 2 387 971 A1**
(43) Date of publication of application: **23.11.2011**
(21) Application number: 09832745.5
(22) Date of filing: 14.12.2009
(51) Int. Cl.: A61F 2/12

(54) **SILICON IMPLANT WITH EXPANDABLE AND/OR INTERACTIVE COMPARTMENTS, OPTIONALLY COATED WITH A RICINUS COMMUNIS AND/OR HYDROXYLAPATITE POLYURETHANE FOAM, WITH ATTACHMENT FLAPS OR STRINGS**

(30) Priority: 19.12.2008 BR PI0805495
(71) Applicant: De Miranda, José Maria, CEP: 24.230-200 - Niterói, Rio de Janeiro (BR)
(72) Inventor: De Miranda, José Maria, CEP: 24.230-200 - Niterói, Rio de Janeiro (BR)
(74) Representative: Hardy, Rosemary
(86) International application number: PCT/BR2009/000410
(87) International publication number: WO 2010/069019

(57) **Abstract**

The invention relates to an implant for augmenting or reconstructing breasts, buttocks, thighs and calves, having independent expandable and interactive compartments that can be filled during manufacture or during or after surgery, with an external silicone membrane (1) internally and/or externally coated with a *Ricinus communis* polyurethane foam (14) covered with hydroxyapatite microcrystals (13) or nanocrystals (13) incorporated into the same membrane, with an inner space divided into compartments (3) that are or can be filled with three-dimensional geometric structures (2) immersed in cohesive silicone (3) or another gel, liquid, air or gas, with a chamber for the projection of the body (4) or of the lower pole of the breast, having a conical structure within the main chamber in order to project the latter. The inner and outer chambers are independently filled and have different volumes, and since they are interactive, they allow any necessary adjustments of the implant through filling valves (8), in order to project the region (6) of the areola and nipple, the body or other region of the breast, correcting ptosis, assymetry, congenital thoracic hypertrophy in the mammal or other region. Flaps (10) or strings (11) for immediate attachment ensure safe and early mobility, faster return to normal activity, and a secure final placement of the implant; they avoid displacement of the implant and can be previously removed by the surgeon.

## Description

This Invention Patent aims to provide an innovative model for silicone implant for breast enlargement or reconstruction; or for other regions of the human body, in order to significantly expand the implant utilization and improve its efficiency. In addition this invention provides technical improvements when compared to existing silicone implant models.

The current implant models used for enlargement or reconstruction of breast, buttocks, thighs and calves, comprises basically a membrane of silicone elastomer graded for medical use. As for all other raw materials graded for medical use this is filled with either a saline solution, silicone gel with varying in cohesion or another product that is biocompatible.

The silicone implant can also present different surface textures such as smooth, with patterns or covered with standard polyurethane foam, not made from *Ricinus communis (castor* bean). All with various volumes and shapes.

Some implants have an inner compartment filled with a more or less consistent cohesive silicone. They can also have an internal compartment that can be filled with saline solution injected through valves coupled or not to the implant. This allows a widespread increase in volume during or after surgery, however it does not allow modelling or increasing the volume at specific regions of the breast that may be desired by surgeons or patients.

Aiming for a shorter fixation of the implant in the human body (avoiding its displacement) the current implants can present either a textured surface or a surface coated with standard polyurethane foam (not from *Ricinus communis).* They can also have small channels on its base.

A wide range of companies is currently producing silicone implants in the world with their quality and prices presenting little variation. These implants often have a series of constraints that are discussed below.

The silicone implant with a membrane coated with standard polyurethane foam has the disadvantage of requiring its peremptorily removal when an infection is resulted from contamination by fungus or bacteria. Otherwise it will be expelled from the body by the antigen-antibody mechanism.

In those circumstances when the silicone implant is not expelled a coarse fibrous capsule is created resulting in a changed texture of the breast region, which is less natural.

Another inconvenience of the capsule is the difficulty for its removal by surgeon because the strong fibrotic adherence to living tissues.

The silicone also known as polymerized siloxane or polysiloxane is a polimer of mixed organic and inorganic materials with chemical formula of [R2SiO]n, where R is the organic group such as methyl, ethyl and phenyl.

It comprises an inorganic silicon-oxygen skeleton (...-Si-0-Si-0-Si-0-...) with the organic side groups attached to the silicon atoms.

By varying the length of the main chain, the type of side groups and the links between chains, silicones can be synthesized with a wide variety of properties and compositions. It can range its consistency from a liquid to gel, rubber or hard plastic.

Because the current silicone implants are made with organic-inorganic materials they can react adversely with the human body. These are known as foreign body reaction, which aims to isolate the body creating a coarse fibrous membrane or capsule (scar) around the implant, resulting in a very artificial appearance and touch.

This is one of the issues most commonly observed after silicone implant surgeries resulting in capsular contraction and hardening of the breast. It occurs when the capsule surrounding the implant begins to push it trying to isolate the body.

In addition, the current silicone implants coated with standard polyurethane foam is likely to bend.

In order to overcome the issues discussed above an innovative model of silicone implant was developed to make implants more secure, fully anatomical, with natural look and light. This is subject of this patent that was developed after more than 20 (twenty) years of careful research and testing.

This innovative silicone implant was designed for using in breasts, buttocks, thighs and calves. It has an internal or external intelligent compartment and can have its texture either smooth, with pattern or coated with polyurethane foam made of *Ricinus communis.* It can also be internally lined with polyurethane foam made of *Ricinus communis,* which is associated with the use of micro or nano crystals of hydroxyapatite.

Firstly, this invention addresses the structural aspect of the silicone implant. It has one standard compartment with internal subdivisions and can also have an external expandable compartment. Both compartments are further illustrated in drawings.

A major concern for potential users of implants and health professionals is its safety.

Concerning its safety, the proposed silicone implant is divided in three parts: (a) the internal wall of the implant is lined with polyurethane foam made of *Ricinus communis* which reduces the risk of rupture and leakage of its contents to almost zero percent; (b) the polyurethane polymer foam made of *Ricinus communis* has no risks of infection owing to its germicide (fungicide and bactericide) action; and (c) no displacement because the presence of appendicular "claws" originated from the edge of the base of the implant.

They may also contain fixing tabs with holes punched in the upper and lower side of the base of the implant.

One or more filling valve can be placed at the bottom tab allowing future filling of each expandable compartment.

The proposed invention is also related to the use of *Ricinus communis* polyurethane as this material interacts better with the human cells decreasing to almost zero the cases of foreign body reaction. This has also the approval of the Federal Drug Administration (FDA), the government agency responsible for North American release of new foods and medicines.

In Brazil, since 1999 the biomaterial was approved by the Health Ministry and during this period many people have benefited from rigid implants for repairing bone tissues.

The great advantage of this polymer is its complete biocompatibility and its fungicide and bactericide effect, which prevents the implant to be expelled even with the presence of infection, avoiding the formation of a coarse or thick capsule.

This polymer is proved to have a high germicidal and sterilization capacity. Produced with different textures and densities, the proposed polymer can replace perfectly the silicone implants, eliminating the risks of allergic reactions or antibody-antigen.

For the patient, the polyurethane foam made of *Ricinus communis* improves the quality of the implant because of its antibacterial and antifungal effect that forms a thinner capsule, and should infection be present. Its removal its not require. Time, overall cost with medication, transportation, fees and beyond lessening the expectation of new surgeries it results in a quicker return of the patient's daily activities.

For the surgeon, the reduced risk of rupture and infection results in a safer surgery and post-surgery; preventing *stress,* legal issues and unpleasant time-consumption.

For the relatives of the patient a safer implant means less concerns, labour and costs.

Another innovation is the finding that the cover (Membrane covering the implant) can be coated with hydroxyapatite micro-crystals attached to its external surface or hydroxyapatite nano-crystals embedded in the silicone membrane of the implant. This offers greater biocompatibility, avoiding the formation of fibrous capsule, leaving a coarser texture to the breast tissue which is similar to the natural aspect.

Laboratory studies assessing biocompatibility of alloplastic materials have proven that using hydroxyapatite in the membrane of the implant has better results over the pure silicone currently used in implants due to it forming a finer fibrous capsule.

For this reason silicone implant coated with hydroxyapatite may be recommended for internal implant in any region of the body, because the membrane surface of the implant does not change the texture of the region surrounding the implant.

As for changes in shape and volume of the implant to correct physical asymmetry (i.e. asymmetry of breast shape and volume when one breast bigger than the other) in regions showing specific variations. This innovative implant allows adjustments in volume and shape because they have more than one independent compartment (internal and / or external). However, they are interactive with the possibility of filling different sectors of the same organ or region therefore shaping it.

Based on the above paragraph, this innovative implant can make the process personalised because each independent compartment can be filled with a liquid substance (saline, distilled water and others), gels (cohesive silicone, gel of *Ricinus communis,* hyaluronic acid, hydroxyapatite, and *others)* or gaseous (air, gases). And also be interactive through injections or remote valves coupled to the implant.

Therefore, they are much safer, smarter and comprehensive. Presenting natural looking results, in compliance to the physician's plan and results desired by the patient.

In case of corrections after the surgery, for example correcting ptosis (drop) of the mammary cone in implants to increase the volume or to improve the ptosis, this implant has independent and interactive slots that can be filled enabling correction of the shape, swelling and ptosis.

In some cases the breast will drop after some time because of gravity and the weakening of the collagen / elastic fibres. Also, the placement of any implant will result in an increased weight of the body which is primarily supported by the skin. Thus, over the years, the breast will continue to drop at a rate proportional to its weight.

The breast increase using traditional implants has very little ability to raise the breast relying more on the volume increase. If the skin flaccidity is small the implant may raise the breast however the same may not happen if significant flaccidity is present. In this case a large implant (in volume) may be required to raise the breast, which may result in a disproportional breast when compared to the chest area.

Using this new model for customized implants, both volume and shape can be changed resulting in a more precise intervention in each region of the body that needs correcting. This will apply to cases such as breast ptosis and flaccidity of small and medium-grade, and correction of asymmetry in shapes or volumes considering especially the disparity between adipose tissue and glandular tissue.

The problem is easily corrected through a controlled process for filling the inner compartment of the papillary-areola complex (areola and nipple) and the external virtual compartment, which prevents an unwanted increase in the overall volume of the implant.

This patent is also based on the innovative system for filling the implant, which will result in significant decrease of the overall weight therefore reducing the effects of the gravity.

The proposed implant will have a main chamber already filled at the factory that can alternatively be filled by the surgeon using 3D structures that are mini geometric structures with variable shapes and dimensions, with a single or multiple chambers. These can have in its core other smaller geometric structures such as small balls (spheres) with silicone walls thinner than the implant surface. The structures will be filled with biocompatible materials such as air, gas, liquid or substances lighter than silicone or other biocompatible gel. This process will minimise risks of overflow and will make the implant lighter (when filled with substance with lower density than the cohesive silicone}, which will reduce the effects of gravity making the implant more compatible with the natural movement in the implanted area.

The new silicone implant for breast, buttocks, thighs and calves with intelligent and interactive compartments solves all possible issues allowing a customised implant with good interaction with human cells reducing the cases of rejection and formation of coarse fibrous capsule.

The invention also brings an innovative tab on the cardinal points for immediate fixation of the implant during surgery and a concentric trench at the base of the implant for fixation in the first weeks following surgery, avoiding the undesirable effects such as displacement of the implant shortly after the surgery.

The tabs (with or without "claws") also allow a shorter recovery post surgery and the patient return to its normal activities. These tabs are optional and can be removed by the surgeon beforehand.

The drawings attached to this report present the main implant and its possible variants as follows:

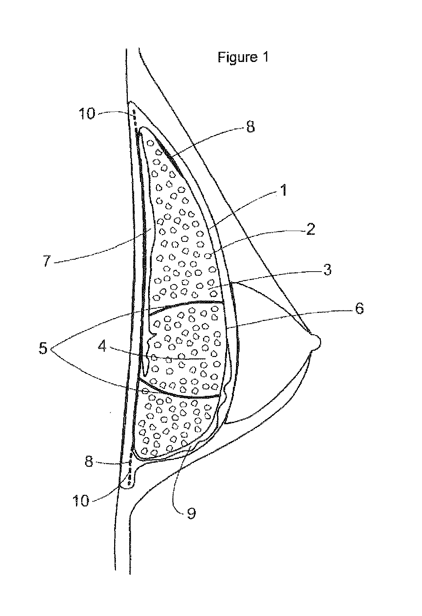

Figure 1 shows the profile of the implant when deflated.

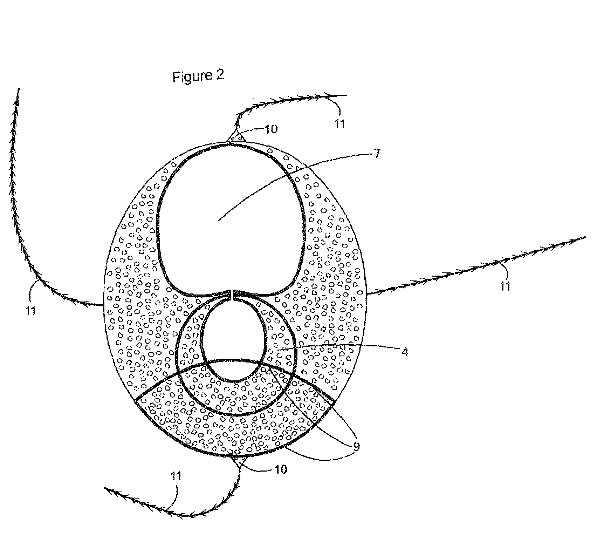

Figure 2 shows the top view of the implant.

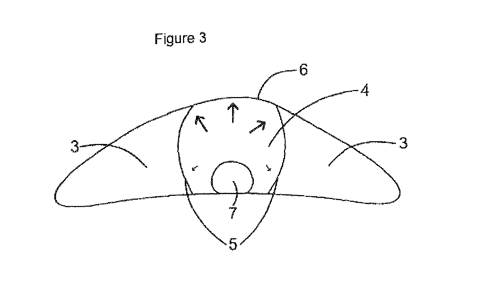

Figure 3 shows the front section of the implant.

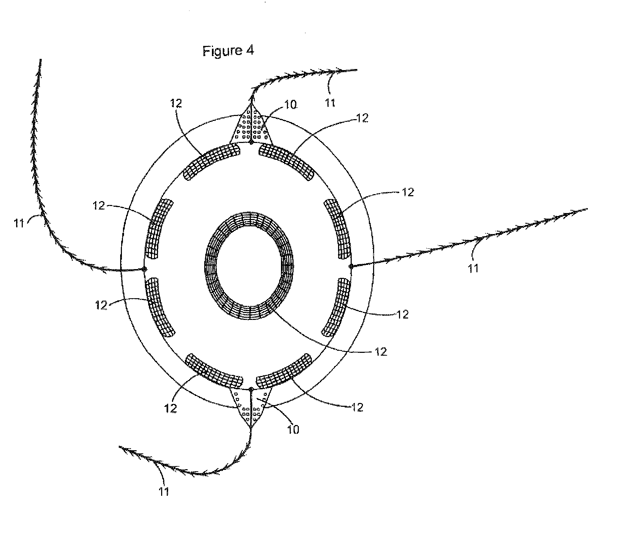

Figure 4 shows the back (base) of the implant

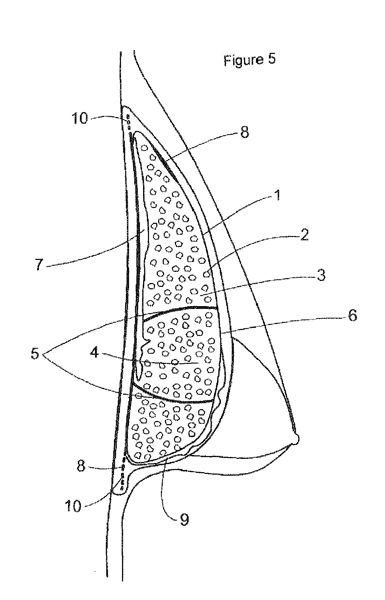

Figure 5 shows the profile of the implant with the consequences of the weight and gravity.

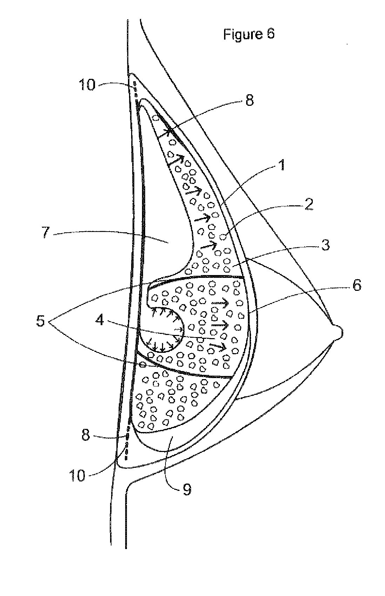

Figure 6 shows the profile of the implant with the projection of the breast (papillary-areola complex) and the compartment already filled.

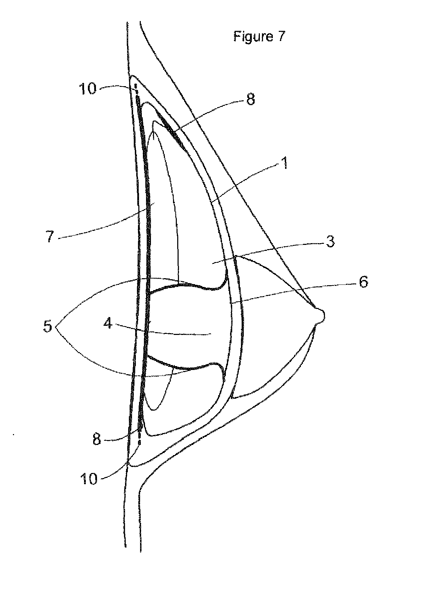

Figure 7 shows a possible structural variation of the chamber and compartments of the implant.

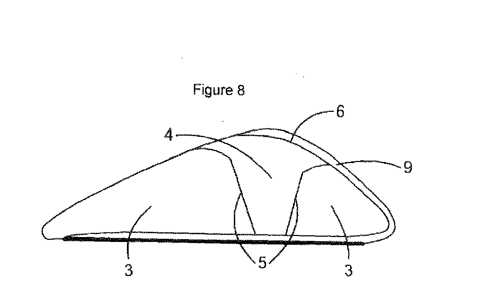

Figure 8 illustrates another possible variation of the structure and compartments of the implant and chamber deflated.

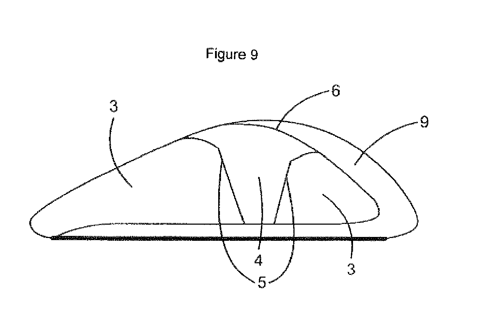

Figure 9 shows the chamber from Figure 8 filled.

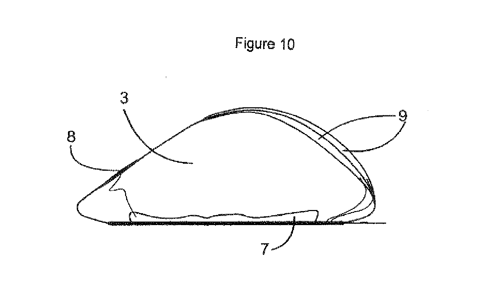

Figure 10 shows another possible arrangement of compartments and chambers.

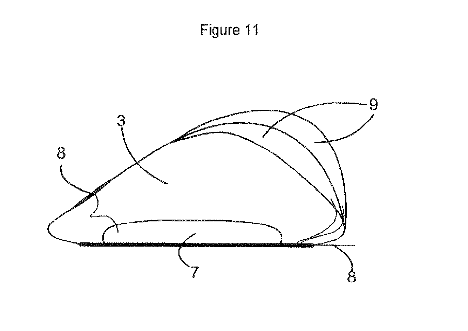

Figure 11 shows the chamber from Figure 10 filled.

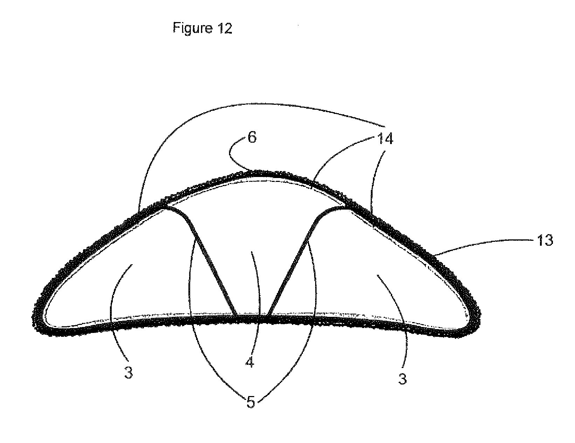

Figure 12 shows the front section of the implant, internally lined with polyurethane foam and micro *Ricinus communis* 14 and hydroxyapatite crystals 13 adhered to the implant texture.

In accordance with the above figures this innovative model of implant subject of this patent consists the following: 1 A silicone implant, 2 filled with geometric structures, 3 embedded in cohesive silicone gel or any other biocompatible material, or only filled with cohesive silicone gel or any other biocompatible gel; 4 has intelligent and interactive compartments with chamber for projection of the breast cone (papillary-areola complex); 5 with a thicker side wall of the cone, 6 to project the top chamber of the breast cone. 7 The walls of the papillary-areola complex are thinner than the implant walls; 8 the interactive chamber allowing customised adjustment through filling valves. 9 Virtual chamber for projection of the breast cone, 10 that can be filled for correction of ptosis, with these walls making the implant look more natural. 11 Tabs for a safer fixation during surgery and to ensure the implant won't move during surgery, which can also be removed by the surgeon prior to surgery if required

Another important factor is the cost of implants coated with polyurethane made of *Ricinus communis.* Since the raw material base for this implant is extracted from castor beans the final cost is 15% to 20% (percent) less than implants coated with standard polyurethane.

The proposed implant can be manufactured in different sizes, models, volumes, textures, and varying in capacity to meet different needs.

## Claims

1. SILICON IMPLANT WITH EXPANDABLE AND/OR INTERACTIVE COMPARTMENT, COATED OR NOT WITH POLYURETHANE FOAM MADE OF RICINUS COMMUNIS AND/OR HYDROXYAPATITE, WITH FIXING TABS OR CORDS, **Characterized by** the fact that the silicone implant is intended to rebuild the breasts and/or increase the breasts, buttocks, thighs and calves with single or multiple compartments, internal or external expandable or not, and when multiple interactive, filled or not factory, or filled by the surgeon at the time or after surgery; a chamber (space) common filled main internal and/or geometrical spacial structures with mini tillable spaces of varying shape and dimensions that may have single or divided chambers or can hold in its core other geometrical structures even smaller space, also of varying shapes and dimensions, such as mini bags (spheres) whose walls are less thick silicone cover the implant completely filled or half filled (amoeboid appearance) with substantial air, gas, liquid or gelatinous (ex: air or helium, saline solution or another liquid biocompatible, non-toxic, or a mixture of biocompatible gases or carboxymethyl or hyaluronic acid, or hydroxyapatite gel or Ricinus communis oil/gel (castor) or polymethylmethacrylate gel or cohesive silicone or the biocompatable combination of these substances. Geometric spacial structures are immersed in cohesive silicone or other gel, liquid, air or gas previously mentioned. These spacial geometric structures could be divided in other internal chambers and each chamber may contain different biocompatable substances. The base of the breast implants present two "gutters" (depression), external and internal in circular form and concentric with up 2 mm depth by 5 mm width "plugged" by polyethylene mesh or other substance nonabsorbable, being the external interrupted by up to 30 mm on the four colateral points, superior and inferior tabs and appendicular cords locaded on the four cardinal points. The external silicone membrane (cap) could be coated or not, internal and/or external with the Ricinus communis polyurethane foam or with hydroxyapatite microcrystals and/or with hydroxyapatite nanocrystals, incorporated to the same membrane, with chambers to protect the mammary cone (areolocapilar region) thicker sidewall with interactive chambers, internal or external, with or without cords appendicular, whether or not absorbable, which can carry or not "claw" (herringbone type). These strings can also be fixable or not by commun surgical suture, these cords that are centrifugal extensions from one or more cardinal points of the edge of the base of the implant. Circular base that has a greater thickness, i.e., higher density, with or without tabs, or not absorbable, with many leaked holes that may or may not port "claws "like on the back to lock in one or more cardinal points of the database.
